(19) Europäisches Patentamt
European Patent Office
Office européen des brevets

(11) **EP 2 688 686 B1**

(12) **EUROPEAN PATENT SPECIFICATION**

(45) Date of publication and mention
of the grant of the patent:
**17.08.2022  Bulletin 2022/33**

(21) Application number: **12713338.7**

(22) Date of filing: **13.03.2012**

(51) International Patent Classification (IPC):
**G01H 11/06** *(2006.01)*     **B06B 1/02** *(2006.01)*
**G10K 11/00** *(2006.01)*

(52) Cooperative Patent Classification (CPC):
**G01H 11/06; B06B 1/0292; G10K 11/002**

(86) International application number:
**PCT/IB2012/051173**

(87) International publication number:
**WO 2012/127360 (27.09.2012 Gazette 2012/39)**

(54) **ULTRASONIC CMUT WITH SUPPRESSED ACOUSTIC COUPLING TO THE SUBSTRATE**

ULTRASCHALL-CMUT MIT UNTERDRÜCKTER AKUSTISCHER KOPPLUNG AN EIN SUBSTRAT

CMUT ULTRASONORE SANS COUPLAGE ACOUSTIQUE AVEC LE SUBSTRAT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **22.03.2011   US 201161466172 P**

(43) Date of publication of application:
**29.01.2014   Bulletin 2014/05**

(73) Proprietor: **Koninklijke Philips N.V.
5656 AG Eindhoven (NL)**

(72) Inventor: **FRASER, John Douglas
NL-5656 AE Eindhoven (NL)**

(74) Representative: **Philips Intellectual Property &
Standards
High Tech Campus 52
5656 AG Eindhoven (NL)**

(56) References cited:
**WO-A2-2010/146838      US-A1- 2004 113 524
US-A1- 2008 048 211      US-A1- 2009 122 651
US-A1- 2011 018 387**

**Description**

This invention relates to medical diagnostic ultrasound systems and, in particular, to cMUT (capacitive micromachined ultrasonic transducer) arrays with suppressed acoustic coupling of reverberation energy to the substrate of the array.

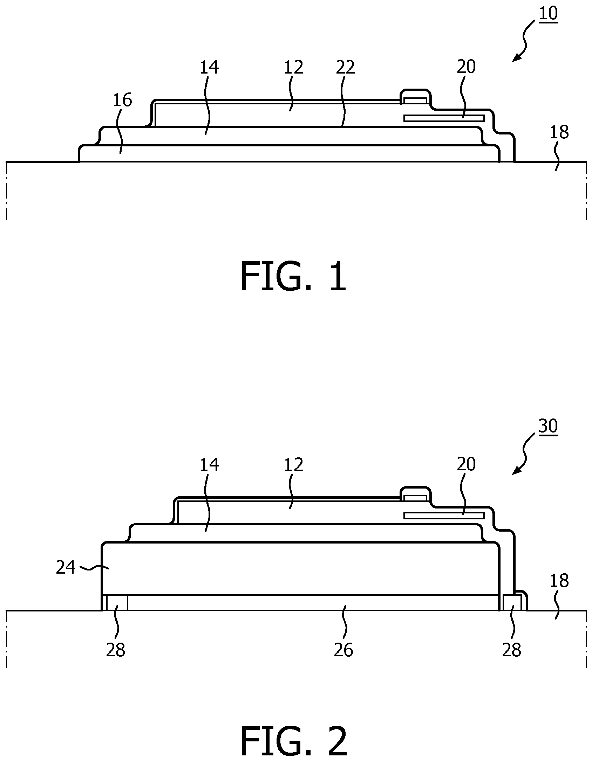

MUTs, and cMUTs in particular, are ultrasonic transducer elements produced by semiconductor fabrication techniques. Unlike conventional piezoelectric materials such as PZT, MUTs may operate by other than strictly piezoelectric effects. In the case of a cMUT, a membrane is vibrated by a variable capacitive effect, in the manner of the diaphragm of a drum. The vibration of the membrane produces the transmitted ultrasonic energy. On reception, the membrane is vibrated by the returning echo and a capacitive variation is sensed to detect the received echo signal. A typical cMUT cell is shown in Fig. 1 of my US Pat. 6,328,697. An electrical schematic for driving a cMUT cell with an a.c. signal at ultrasonic frequencies is shown in Fig. 2 of this patent.

When the membrane of the cMUT vibrates to transmit ultrasonic waves, the force of the vibration is supported, in accordance with Newton's third law, by the substrate on which the cMUT is fabricated. Known cMUT elements, according to Newton's third law, apply equal and opposite mechanical forces to their supporting substrates in relation to the acoustic pressure forces applied to the load medium in the desired direction of transmission. Furthermore, cMUT arrays, due to their periodic structure and construction with a support ring holding each top membrane separated from the substrate, and sometimes with a collapsed region in the center, apply this average force in a periodic way across the array. Forces applied to the substrate, typically a very low acoustic loss material such as silicon, generate one or more of several acoustic wave types such as longitudinal waves, shear waves, Lamb waves, and Rayleigh waves, which may propagate through the substrate and laterally along the substrate. In any of these cases, the results are similar. The waves carry energy in the substrate, which is received by reciprocal mechanisms of other cMUT elements on the substrate and interpreted by them as if an incoming signal, but after an inappropriate and sometimes very long time relative to the desired signals. This causes spurious electrical signals to be generated and interpreted by the attached imaging system as incoming signals, creating artifacts in the generated image. Acoustic coupling is so favorable, and acoustic losses so low in silicon, for example, that clutter in the image can decrease contrast severely. Energy transferred from one array element to neighboring elements can decrease the array acceptance angle sufficiently to seriously compromise lateral resolution and beam steering capabilities. These issues have contributed to the lack of commercial acceptance of cMUTs in the medical imaging field.

The prior art illustrates various attempts at preventing acoustic coupling into and through the MUT substrate. These efforts include the use of matched acoustic backing behind the substrate as illustrated by, *inter alia,* US pat. 6,862,254, US pat. 6,831,394, and US pat. 7,441,321, which tries to deaden unwanted acoustic energy from behind the substrate. Another approach is the thinning of the substrate as illustrated in US pat. 6,714,484 and US pat. 6,262,946, which attempts to prevent the travel of waves laterally along the substrate by removing the substrate to as great a degree as possible. Yet a further approach is the inclusion of various types of discontinuities in the substrate to scatter or block the propagation of spurious waves through the substrate as shown in US pat. appl. pub. US2009/0122651, US pat. 7,741,686, US pat. 7,545,075, and US pat. 6,669,644. What is needed is a simple and effective way to prevent the transmission of energy into the substrate which is the source of the unwanted artifacts.

US 2011/018387 A1 discloses a transducer having a membrane, upper electrodes, cavities, a lower electrode and a circuit board. A silicon substrate has grooves and couples the circuit board to the other elements.

WO 2010/8146838 A1 discloses a transducer having a vibrating membrane, an upper electrode, gap, a lower electrode and a circuit board. A wiring line substrate connects the circuit board to the other elements.

US 2008/048211 A1 discloses a CMUT having a membrane, a back electrode, cell cavities, and a circuit board.

US 2009/0122651 A1 describes a transducer having a membrane layer, a top electrode, a cavity, a bottom electrode and an electronic unit. A silicon layer acts to connect the electronic unit to the other components.

According to an aspect of the inventive concept, there is provided an array of cMUT cells according to claim 1. The dependent claims provide optional embodiments.

The invention provides for an array of cMUT cells of an ultrasound transducer according to independent claim 1. Preferred embodiments of the invention are provided for in the dependent claims.

In accordance with the principles of the present invention, a MUT array is provided with MUT elements acoustically isolated from the substrate. The acoustic force of transmission of a MUT element is opposed by a relatively significant mass which supports the MUT element. The support mass is mounted on the substrate by a plurality of support members of small size and/or low stiffness which provide low coupling from the support mass to the substrate.

In the drawings:

FIGURE 1 illustrates a typical prior art cMUT cell in cross-section.

FIGURE 2 is a cross-sectional view of a cMUT device which is acoustically isolated in accordance with the principles of the present invention.

FIGURE 3a is a schematic illustration of the coupling physics of a cMUT device of the present invention.

FIGURE 3b is an exploded view of the schematic illustration of FIGURE 3a illustrating the forces involved in operation of the device.

FIGURE 4 is a cross-sectional view of another MUT device which is acoustically isolated in accordance with the principles of the present invention.

FIGURE 5 is a cross-sectional view of another MUT device which is acoustically isolated in accordance with the principles of the present invention.

FIGURE 6 is a plan view of an array of hexagonal cMUT cells constructed in accordance with the present invention and illustrating electrical connections to the cells.

FIGURE 7 is a plan view of an alternate technique for making electrical connections to an array of cMUT cells in accordance with the present invention.

FIGURE 8 is a cross-sectional view of a cMUT fabricated on a semiconductor substrate with ASIC circuitry for operating the cMUT in accordance with the principles of the present invention.

Referring first to FIGURE 1, a typical cMUT device 10 of the prior art is illustrated in cross-section. The cMUT 10 includes a top electrode 12 made of an electrically conductive material. The top electrode is located on a membrane 22, or may itself comprise the membrane. In this illustration the membrane is formed of a nonconductive material such as silicon nitride or silicon dioxide. The membrane is supported by vertical supports at its lateral edges over a void or gap 14. In this embodiment the membrane spans across the gap without touching the floor at the bottom of the gap. In other embodiments the membrane may be purposely built or biased to operate in a collapsed mode where the center of the membrane is in contact with the floor of the gap. A conductor 20 couples electrical signals to and from the top electrode 12. Below the gap 14 is a bottom electrode 16. Electrical connections to the bottom electrode are made from the semiconductor substrate 18 on which the cMUT cells of the array transducer are fabricated. The other dark layers in this embodiment are isolation layers, typically formed of silicon nitride or silicon dioxide.

As the top electrode 12 and membrane 22 oscillate when driven by a transmit signal, the desired acoustic signal is transmitted upward from the top surface of the top electrode. But the counter-forces to this force, the resistance to the force of the acoustic pressure wave by the substrate platform on which the cMUT cells are fabricated, cause acoustic waves to be coupled into the substrate 18 where they can travel backward through the substrate and be reverberated back into the cMUT cell where they cause clutter. Unwanted acoustic waves can also travel laterally to adjacent cMUT cells. The lateral waves can reach other cMUT cells during signal reception and be erroneously sensed as received echo signals by those cells. These unwanted signals from the substrate can be interpreted as clutter signals, degrading the quality of the resultant ultrasound image.

FIGURE 2 illustrates in cross-section a cMUT cell constructed in accordance with the principles of the present invention. Conceptually, a cMUT cell of the present invention can be constructed as a normal cMUT but with the addition of a significant amount of mass below the moving membrane, either as part of the lower electrode or attached to it. This mass can take the form of a plate of a very dense material of sufficient thickness to provide substantial reaction to the applied acoustic forces with significantly less motion than would be present in the substrate if the cell was directed mounted on the substrate. The massive plates for individual cells or groups of cells are laterally acoustically isolated from each other to prevent lateral coupling from one massive plate to another. The massive plate is preferably suspended above the substrate by small supports such as small posts of minimal cross-sectional area to further reduce acoustic coupling into the substrate. In a further embodiment the massive plate can be suspended on small supports, with the space between the plate and the substrate adjacent to the supports filled in with a compliant material such as polydimethyl siloxane (PDMS), also known as silicone rubber.

In the example of FIGURE 2, the top electrode 12 is a conductor such as aluminum, tungsten, a polysilicon membrane, or single crystal silicon. The top electrode 12 is compliant and also operates as the membrane of the cMUT device. Electrical connection to the top electrode 12 is made by a conductor 20 formed, for example, of tungsten, aluminum, copper, or polysilicon. The top electrode 12 may typically be 1-5 microns thick with a diameter across the electrode of 30-100 microns. The shape of the cMUT cells can be circular or other shapes such as hexagonal, rectangular, or square.

A gap 14 is located between the top electrode 12 and a massive plate 24. The massive plate 24 is formed to have a high stiffness at the frequencies and thicknesses of interest. The plate 24 will then be considered small, e.g., one-tenth or less, compared to a wavelength of any important acoustic propagation mode at which the cMUT cell operates. For example, the mass and stiffness requirements can lead to use of a material having a high acoustic impedance such as an acoustic impedance greater than 40 MegaRayls (MRayl). Suitable materials for the massive plate include tantalum (55 MRayl) gold (64 MRayl), molybdenum (63 MRayl), tungsten (101 MRayl), copper (42 MRayl) or chromium (43 MRayl), as well as alloys of these materials. One practical material would be a titanium-tungsten alloy, which is readily available in most semiconductor fabs. The choice of an electrically conductive material such as tungsten enables the massive plate 24 to additionally serve as the bottom electrode of the cMUT.

The massive plate 24 is not fabricated directly on the substrate 26 but is supported by several end posts or edge supports 28. These small posts 28 are made of materials available in the semiconductor fabrication process such as silicon, silicon nitride, or silicon oxide. Conductive materials may also be used if appropriately electrically isolated. A typical height of the posts is 3 microns. The posts should be sufficient to resist static applied forces that would otherwise deform the massive plate, yet be small enough that the total stiffness added to support the plate is small compared to the inertial resistance supplied by the mass of the plate itself at acoustic frequencies of interest. Between the posts 28 is a second gap 26. This gap may be filled with a vacuum, open to the air, or filled with a compliant material such as silicone rubber (PDMS). By filling the second gap with a compliant material such as PDMS, contamination of the space with unwanted substances is avoided. An array of cMUT cells such as the one shown in FIGURE 2 can be fabricated by a process based on the deposition of layers and sacrificial etching. The devices can also be made by wafer bonding techniques or a combination of these processes.

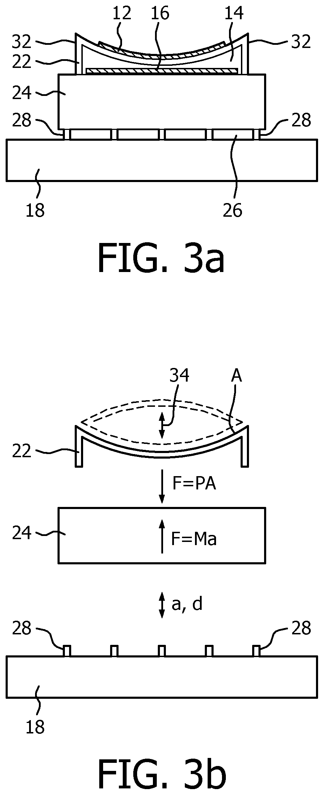

FIGURES 3a and 3b illustrate the inventive concept of the present invention. FIGURE 3a schematically illustrates the elements of the cMUT of FIGURE 2 stacked in the same configuration. The membrane 22 is supported for oscillation by lateral supports 32 and is mounted on the massive plate 24 in FIGURE 3a. A top electrode 12 is located on top of the membrane and a bottom electrode 16 is located below the membrane. The massive plate 24 is supported on the substrate 18 by a plurality of small posts 28, which are separated by the spaces of lower gap 26.

FIGURE 3b shows an exploded view of this assembly and the acoustic forces involved in operation of the cMUT. The membrane 22 oscillates up and down during ultrasound transmission as indicated by arrow 34 and the curves above and below it. As the membrane oscillates it generates the pressure force of the transmitted sound wave with a pressure P. This pressure is exerted by the membrane area A, and consequently a force is developed which can be calculated as $F=PA$. The force of this pressure wave is directed downward through the lateral supports of the membrane. The body on which the supported membrane is mounted, in this case the massive plate 24, opposes the acoustic pressure force generated by the moving membrane. It does this with the inertia of its mass. This opposing force is $F=Ma$, composed of the larger mass M of the massive plate and the acceleration "a" associated with its oscillatory motion. As the two forces are equal and opposite and the plate is massive, the result is that the motional component of the massive plate exhibits a much smaller acceleration and movement, represented in the equation by "a". Thus, the pressure force of the cMUT is not resisted by a large motion component in the substrate, but only a much smaller motion "d" associated with the acceleration "a" which must be contained. This is done in FIGURES 3a and 3b by supporting the massive plate 24 on a number of uniformly distributed small posts 28 of compliant material, which act to further attenuate the transmission of motional force into the substrate 18 by responding to the motion "d" with a force F' much smaller than the force F described above. In this way the large inertial resistance of the massive plate largely prevents the transmission of unwanted motion at acoustic frequencies into the substrate.

An example with calculations for typical materials demonstrate how the performance of the cMUT cell isolation system can be calculated and the desired dimensions and properties of the massive plate and second gap filler can be determined. Assume, for example, that the cMUT cell, in transmission, generates a sound field of 1 MPa peak pressure level in a load medium equivalent to water, with acoustic impedance of 1.5MRayl, oscillating at a frequency f = 20 MHz, typical parameters for catheter-based imaging. If the cell has an area A, then the reaction force on the front surface of the cell is

$$1 \text{ MPa x A,}$$

which would be applied directly to the substrate in a traditional cMUT assembly. The average motion amplitude at the front surface of the transducer is

$$1\text{MPa}/(\,2*\pi*f*1.5\text{MRayl}) => 5\text{nm.}$$

The mass of the reaction plate is determined by its density, thickness, and area (generally about the same as the area of the cMUT cell). High density material is preferred for the massive plate because a smaller thickness of material is then required, simplifying the semiconductor processing. In this example Tungsten is chosen for the plate material. Now if we consider a 3um thick layer of Tungsten acting as a massive plate, the mass per unit area is given by density times thickness,

$$19300 \text{ kg/m}^3 * 3*10^{-6}\text{m} => 58 \text{ g/m}^2,$$

and the resulting motion amplitude d of the plate, neglecting reaction from the compliant materials below it, is obtained from F=Ma, which for harmonic motion at a frequency f is given by

$$d=a/(4\pi^2 f^2),$$

and so

$$d=F/(4*\pi^2*f^2) =0.06nm.$$

While the space between the massive layer and the substrate may be evacuated or air-filled, for ruggedness in manufacturing and use it is desirable to fill this gap with a soft solid material. Although the acoustic isolation with vacuum or air might be somewhat better, commonly available PDMS rubber is an acceptable choice. We may calculate the average pressure applied to the substrate by this motion through, for example, a 3um layer of PDMS rubber with an acoustic impedance $Z_a$ of 1MRayl and a speed of sound $v_a$ of 1000 m/sec.

The induced strain in the PDMS is the deformation divided by the thickness, in this case 0.06nm/3000nm = $2*10^{-5}$, where the stiffness of the PDMS is so low as to not affect the amplitude of the motion of the massive plate significantly, and the longitudinal stiffness is

$$c_{11}=Z_a*v_a= 1GPa.$$

Therefore the stress, or pressure on the substrate is

$$P = c_{11}*S = 2*10^4 Pa,$$

which is a factor of 50 in amplitude smaller than the 1MPa which would occur without the present invention. This level of performance results in a 34dB attenuation of the substrate excitation force below that applied to the load medium by the cMUT. Different levels of performance may be desirable in other implementations. For example, attenuation levels of 50% (6dB), 66.67% (10dB), or 90% (20dB) of the acoustic force on the substrate may be acceptable levels of performance in other embodiments of the present invention.

As long as the support structures occupy no more than about 1/50th of the surface area of comparable stiffness to the massive layer, or a value which can be considerably more if the support structures can be derated for their compliance, this level of substrate coupling performance can be expected. If a solid compliant layer is applied between the massive layer and the substrate, then the use of compliant supports is preferred, so that the acoustic force applied to the substrate will be uniformly applied over the entire surface under the cMUT cell by the solid layer, to decrease the likelihood of generating laterally propagating waves due to laterally periodic excitation through the support structures.

For the alternate case of a vacuum gap and multiple small support posts, we may do a similar calculation. Given a 3um gap with 0.06nm of motion at the top surface, and a desire to limit the transferred force to $2*10^4$ Pa, with a post material of stiffness $c_{11}$ = 290 GPa (typical for silicon nitride),

$$P = c_{11}*S*A_f = 2*10^4 Pa$$

where $A_f$ is the fraction of the surface area comprising posts, then

$$A_f = 2*10^4 Pa / (290*10^9 Pa * 2*10^{-5}) \cong 0.3\%$$

For a circular 30um diameter cMUT cell, for example, this requirement would be satisfied with three 1um diameter straight cylindrical posts, which would be marginal from a static support point of view. If a less stiff material were used, more posts could be provided. For example, with silicon dioxide at a stiffness of 80 GPa, with similar dimensions, more than 20 posts would be allowable, well more than needed.

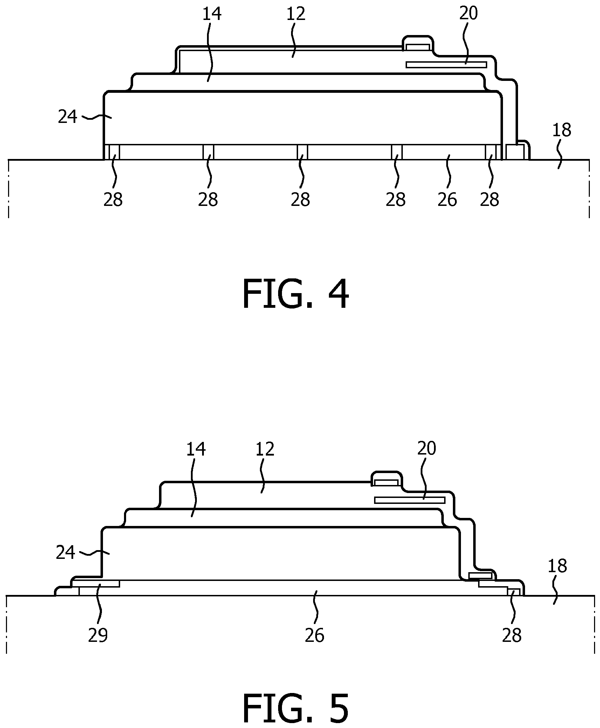

Another example of a cMUT cell constructed in accordance with the principles of the present invention is shown in FIGURE 4. In this example the cMUT with its massive plate 24 is supported by an array of multiple small posts 28 of structural material, which may be any of the materials already in use in the fabrication process, such as silicon, silicon nitride, silicon oxide, or any of a variety of conductive materials, as long as any electrical constraints are met. In order for these posts to mechanically support the device, they should be numerous enough that they can resist the static air pressure load and evenly resist any externally applied static forces that would otherwise deform the massive plate. An example of such a force is the one that would result from the choice to use a vacuum in the gaps 26 between the posts 28. The posts should be small enough that the total stiffness added to the support of the plate is small compared to the inertial resistance supplied at the acoustic frequencies of interest by the mass of the plate itself. Preferably the posts should be arrayed so as to approximately uniformly distribute the support for the cMUT and plate on the underlying substrate 18.

Another example of a cMUT cell constructed in accordance with the principles of the present invention is shown in FIGURE 5. In this example the cMUT with its massive plate 24 is supported by compliant supports 29, such as a ring support in the case of a circular cMUT, around the periphery of the massive plate 24. In the illustrated example the compliant supports 29 provide a compliant cantileverlike support, with the compliant supports 29 in turn supported by a base ring or array of posts 28. The small motional effects not fully eliminated by the use of the massive plate 24 are dampened by the compliance of the support or supports 29.

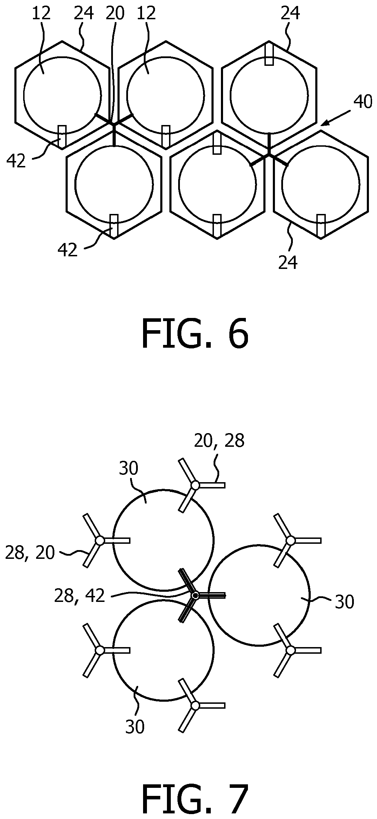

FIGURE 6 is a top plan view of an array of cMUT cells in which each cMUT is circular and the massive plates 24 for the cells have a hexagonal shape. Each cMUT is mounted on its own separate plate 24 and the plates 24 are laterally isolated from each other by gaps 40 between the plates. When the cells are of a shape which has distinctive corners, it is often desirable to fabricate the electrical connections to the cell electrodes at the corners. In this example the top electrodes 12 of the cMUT cells are coupled to a reference potential or ground by corner connections 20. A single connection 20 is seen to branch to connect three cells at their corners in this example. The bottom electrode (16 or 24) in this example is designated as the signal electrode. Connections are made at other corners of the cells to make signal connections 42 to the bottom electrodes of the cMUT cells.

FIGURE 7 is a top plan view of an array of cMUT cells 30 in which each cMUT is circular and the massive plates 24 for the cells are also circular and of the same size as the top electrode or membrane 12 or 22. Each cMUT cell and its massive plate are supported on the substrate by three supports 28, which also carry electrical connections to the cMUT electrodes. In the illustrated configuration, the supports 28 branch in three directions so as to support three different cMUTs 30. The supports designated 20,28 carry the reference potential or ground electrical connections to the top electrodes 12 of the cMUTs. The center support designated 28,42 also is seen to support three cMUTs, and carries individual signal conductors to the bottom electrodes 16,24 of the three cMUTs shown in the drawing.

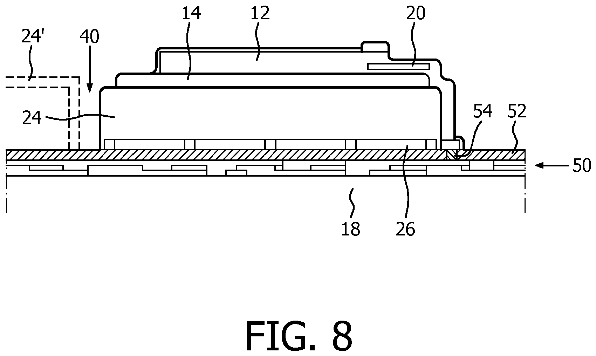

FIGURE 8 is a partially cross-sectional illustration of an array of cMUTs fabricated in accordance with the present invention. In this illustration a layer 50 of integrated circuit components and connections is formed on an IC substrate 18. An isolation layer 52 is laid over the integrated circuit layer 50 and the cMUT array is formed on the isolation layer 52 rather than directly on the substrate 18. Electrical connections are made from the integrated circuitry of layer 50 through the isolation layer 52 to the cMUT electrodes, such as the electrical connection 54 to the conductor 20 of the cMUT. The massive plate 24' of an adjacent cMUT of the array is shown in phantom on the left side of the drawing, separated from the cMUT in the center of the drawing by a gap 40, which may be air-filled or filled with the material normally used for covering the transducer array for wear resistance, acoustic coupling to the load medium, and/or focusing, typically a silicone rubber composite. It is seen that cMUT cells supported by massive plates for motion isolation can be fabricated on the same substrate and in a common semiconductor process as the ASIC circuitry 50 which operates and is responsive to signals received by the cMUT transducer cells.

## Claims

1. An array of cMUT cells of an ultrasound transducer comprising:

a plurality of cMUT cells, each cell comprising:

a cell membrane (22);
a membrane support structure;
a top electrode (12) coupled to the cell membrane (22);
a void (14) providing a space in which the cell membrane (22) moves;
a bottom electrode (16) used in conjunction with the top electrode (12), wherein each cell membrane (22) is adapted to oscillate to thereby generate an acoustic pressure force;

a substrate (18);
wherein each cMUT cell further comprises
a respective massive plate (24) mounted on the substrate (18), wherein:

the cell membrane (22),

the membrane support structure, the top electrode (12) and the void (14) are mounted on the massive plate (24),

the bottom electrode (16) is either formed by the massive plate (24) or is mounted on the massive plate (24),

each massive plate (24) has a mass, the inertia of the mass opposing the acoustic pressure force generated by the oscillation of the cell membrane (22), and

each massive plate (24) is laterally isolated from adjacent massive plates (24) by a gap (40),

**characterized in that** each cMUT cell further comprises a plurality of spaced-apart supports (28), wherein the massive plate (24) is mounted on the substrate (18) by the plurality of spaced-apart supports (28).

2. The array of cMUT cells of Claim 1, wherein the cell membrane, the membrane support structure, the top electrode, the bottom electrode, and the massive plate are fabricated by a semiconductor fab processes.

3. The array of cMUT cells of Claim 2, wherein the massive plates are formed of an electrically conductive material so as to also provide the bottom electrode.

4. The array of cMUT cells of Claim 3, wherein the electrically conductive material is tantalum, gold, molybdenum, copper, chromium, or tungsten or an alloy thereof.

5. The array of cMUT cells of Claim 1, wherein each massive plate further exhibits an acoustic impedance greater than 40 MRayls.

6. The array of cMUT cells of Claim 1, wherein each spaced-apart support is made of a compliant material.

7. The array of cMUT cells of Claim 1, wherein spaces between the spaced-apart supports are filled with one of a vacuum, air, or a compliant material.

8. The array of cMUT cells of Claim 7, wherein the compliant material is silicone rubber.

9. The array of cMUT cells of Claim 8, wherein the each cMUT cells and massive plates exhibit a hexagonal pattern.

10. The array of cMUT cells of Claim 8, wherein the each cMUT cells and massive plates exhibit a circular pattern.

11. The array of cMUT cells of Claim 8 further comprises an integrated circuit layer, wherein the substrate is a semiconductor substrate overlaying the integrated circuit layer.

12. The array of cMUT cells of Claim 1, wherein the acoustic pressure force developed by the cMUT is attenuated by at least 6dB.

13. The array of cMUT cells of Claim 1, wherein the acoustic pressure force developed by the cMUT is attenuated by at least 10dB.

14. The array of cMUT cells of Claim 1, wherein the acoustic pressure force developed by the cMUT is attenuated by at least 20dB.

**Patentansprüche**

1. cMUT-Zellen-Array eines Ultraschallwandlers, das Folgendes umfasst:

eine Vielzahl von cMUT-Zellen, wobei jede Zelle Folgendes umfasst:

eine Zellmembran (22);

eine Membranstützstruktur;

eine Top-Elektrode (12), die mit der Zellmembran (22) gekoppelt ist;

einen Leerraum (14), der einen Raum bereitstellt, in dem sich die Zellmembran (22) bewegt;

eine Bodenelektrode (16), die in Verbindung mit der Top-Elektrode (12) verwendet wird, wobei jede Zellmembran (22) dazu angepasst ist zu oszillieren, wodurch eine Schalldruckkraft erzeugt wird;
ein Substrat (18);

wobei jede cMUT-Zelle weiter eine jeweilige massive Platte (24) umfasst, die auf das Substrat (18) montiert ist, wobei:

die Zellmembran (22), die Membranstützstruktur, die Top-Elektrode (12) und der Leerraum (14) auf die massive Platte (24) montiert sind, die Bodenelektrode (16) entweder von der massiven Platte (24) gebildet wird oder auf der massiven Platte (24) montiert ist, wobei jede massive Platte (24) eine Masse aufweist, die Trägheit der Masse sich der akustischen Schalldruckkraft, die von der Oszillation der Zellmembran (22) erzeugt wird, widersetzt,
und
jede massive Platte (24) seitlich von angrenzenden massiven Platten (24) durch einen Spalt (40) getrennt ist, **dadurch gekennzeichnet, dass** jede cMUT-Zelle weiter eine Vielzahl beabstandeter Stützen (28) umfasst, wobei die massive Platte (24) auf das Substrat (18) durch eine Vielzahl beabstandeter Stützen (28) montiert ist.

2. cMUT-Zellen-Array nach Anspruch 1, wobei die Zellmembran, die Zellstützstruktur, die Top-Elektrode, die Bodenelektrode und die massive Platte durch einen Halbleiterherstellungsprozess hergestellt sind.

3. cMUT-Zellen-Array nach Anspruch 2, wobei die massiven Platten aus einem elektrisch leitfähigen Material gebildet sind, um auch die Bodenelektrode bereitzustellen.

4. cMUT-Zellen-Array nach Anspruch 3, wobei das elektrisch leitfähige Material Tantal, Gold, Molybdän, Kupfer, Chrom oder Wolfram oder eine Legierung daraus ist.

5. cMUT-Zellen-Array nach Anspruch 1, wobei jede massive Platte weiter eine akustische Impedanz größer als 40 MRayl darlegt.

6. cMUT-Zellen-Array nach Anspruch 1, wobei jede beabstandete Stütze aus einem nachgiebigen Material hergestellt ist.

7. cMUT-Zellen-Array nach Anspruch 1, wobei Räume zwischen den beabstandeten Stützen mit einem von Vakuum, Luft oder einem nachgiebigen Material gefüllt sind.

8. cMUT-Zellen-Array nach Anspruch 7, wobei das nachgiebige Material Silikonkautschuk ist.

9. cMUT-Zellen-Array nach Anspruch 8, wobei jede cMUT-Zelle und die massiven Platten eine hexagonale Struktur darlegen.

10. cMUT-Zellen-Array nach Anspruch 8, wobei jede cMUT-Zelle und die massiven Platten eine kreisförmige Struktur darlegen.

11. cMUT-Zellen-Array nach Anspruch 8, das weiter eine integrierte Schaltungsschicht umfasst, wobei das Substrat ein Halbleitersubstrat ist, das die integrierte Schaltungsschicht überlagert.

12. cMUT-Zellen-Array nach Anspruch 1, wobei die akustische Schalldruckkraft, die von den cMUT entwickelt wird, um mindestens 6 dB gedämpft ist.

13. cMUT-Zellen-Array nach Anspruch 1, wobei die akustische Schalldruckkraft, die von den cMUT entwickelt wird, um mindestens 10 dB gedämpft ist.

14. cMUT-Zellen-Array nach Anspruch 1, wobei die akustische Schalldruckkraft, die von den cMUT entwickelt wird, um mindestens 20 dB gedämpft ist.

**Revendications**

1. Un réseau de cellules cMUT d'un transducteur à ultrasons comprend:

   une pluralité de cellules cMUT, chaque cellule comprend:

   une membrane cellulaire (22);
   une structure de support de membrane;
   une électrode supérieure (12) couplée à la membrane cellulaire (22);
   un vide (14) fournissant un espace dans lequel la membrane cellulaire (22) se déplace;
   une électrode inférieure (16) utilisée en liaison avec une électrode supérieure (12), où chaque membrane cellulaire (22)
   est adaptée pour osciller pour ainsi
   générer une force de pression acoustique;
   un substrat (18);

   où chaque cellule cMUT comprend également une plaque massive correspondante (24) montée sur le substrat (18), où:

   la membrane cellulaire (22),
   la structure de soutien de la membrane,
   l'électrode supérieure (12) et le vide (14) sont montées sur la plaque massive (24), l'électrode inférieure (16) est soit formée par la plaque massive (24) ou est
   montée sur la plaque massive (24),
   chaque plaque massive (24)
   possède une masse, l'inertie de la masse opposant la force de pression acoustique générée par l'oscillation de la membrane cellulaire (22), et chaque plaque massive (24)
   est isolée latéralement à partir de plaques massives adjacentes (24) par un espace (40),
   **caractérisé par le fait que** chaque cellule cMUT comprend également une pluralité de supports espacés les uns des autres (28), où la plaque massive (24) est montée sur le substrat (18) par la pluralité des supports espacés les uns des autres (28).

2. Le réseau de cellules cMUT de la revendication 1, où la membrane cellulaire, la structure de soutien de membrane, l'électrode supérieure, l'électrode inférieure, et la plaque massive sont fabriquées par des processus de fabrication de semi-conducteurs.

3. Le réseau de cellules cMUT de la revendication 2, où les plaques massives sont formées à partir d'un matériau électriquement conducteur afin de fournir également l'électrode inférieure.

4. Le réseau de cellules cMUT de la revendication 3, où le matériau électriquement conducteur est du tantale, de l'or, du molybdène, du cuivre, du chrome, du tungstène ou un alliage faisant partie de ces matériaux.

5. Le réseau de cellules cMUT de la revendication 1, où chaque plaque massive présente en outre une impédance acoustique plus forte que 40 rayl.

6. Le réseau de cellules cMUT de la revendication 1, où chaque support espacé est fait d'un matériau conforme.

7. Le réseau de cellules cMUT de la revendication 1, où les espaces entre les supports espacés sont remplis avec un des éléments suivants, le vide, l'air ou un matériau souple.

8. Le réseau de cellules cMUT de la revendication 7, où le matériau souple est du caoutchouc de silicone.

9. Le réseau de cellules cMUT de la revendication 8, où chaque cellule cMUT et des plaques massives présentent un motif hexagonal.

10. Le réseau de cellules cMUT de la revendication 8, où chaque cellule cMUT et des plaques massives présentent un motif circulaire.

**11.** Le réseau de cellules cMUT de la revendication 8, comprend également une couche de circuit intégré, où le substrat est un substrat semi-conducteur recouvrant la couche de circuit intégré.

**12.** Le réseau de cellules cMUT de la revendication 1, où la force de pression acoustique développée par le cMUT est atténuée d'au moins 6 dB.

**13.** Le réseau de cellules cMUT de la revendication 1, où la force de pression acoustique développée par le cMUT est atténuée d'au moins 10 dB.

**14.** Le réseau de cellules cMUT de la revendication 1, où la force de pression acoustique développée par le cMUT est atténuée d'au moins 20 dB.

FIG. 1

FIG. 2

FIG. 3a

FIG. 3b

FIG. 4

FIG. 5

FIG. 6

FIG. 7

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6328697 B
- US 6862254 B
- US 6831394 B
- US 7441321 B
- US 6714484 B
- US 6262946 B
- US 20090122651 A
- US 7741686 B
- US 7545075 B
- US 6669644 B
- US 2011018387 A1
- WO 20108146838 A1
- US 2008048211 A1
- US 20090122651 A1